# EUROPEAN PATENT APPLICATION

(11) **EP 1 103 615 A1**
(43) Date of publication of application: **30.05.2001**
(21) Application number: 99123498.0
(22) Date of filing: 25.11.1999
(51) Int. Cl.: C12N 15/63, C12N 15/64, C12N 5/16, C12N 5/22, C12N 7/01, C12P 21/00

(54) **Vectors capable of immortalizing non-dividing cells and cells immortalized with said vectors**

(71) Applicant: UNIVERSITE DE GENEVE, CH-1211 Geneve 4 (CH)
(72) Inventor: Occhidoro, Teresa, 3057 Melbourne, Victoria (AU); Salmon, Patrick, 74100 Vetraz-Monthoux (FR); Trono, Didier, 1245 Collonge-Bellerive (CH)
(74) Representative: Dufresne, Guillaume Alain François

(57) **Abstract**

Vectors capable of stably integrating a transgene in the genome of a non-dividing cell or of a slowly-dividing cell, said vector comprising or expressing at least one immortalization molecule and cells immortalized with said vectors.

## Description

There is a long-felt need in the art for a method to expand non-dividing or slowly-dividing cells by immortalization because non-dividing or slowly-dividing cells are the predominant, long-lived cell type in the body, and account for most desirable targets of gene transfer, including liver, muscle, and brain.

Most of the immortalization studies carried out in the art have used oncoretroviruses. However, simple retroviruses like oncoretroviruses cannot immortalize non-dividing or slowly-dividing cells because these retroviruses require the dissolution of the nuclear membrane at mitotic prophase to transfer a gene into the genome of a target cell, most likely because the bulky size of the preintegration complex of these retroviruses prevents its passive diffusion through the nuclear pores.

Thus, cell division is necessary for the proviral integration of these vectors.

The invention provides for the first time a vector capable of immortalizing a non-dividing or slow-dividing cell.

In contrast to known delivery systems, a vector of the invention is capable of stably integrating at least one immortalization molecule in the genome of a non-dividing or slow-dividing cell.

A vector of the invention bears at least one or any one of the possible combinations of the following features :
- is capable of stably integrating a transgene in the genome of a non-dividing cell or of a slowly-dividing cell, said vector comprising or expressing at least one immortalization molecule ;
- is capable of transporting a transgene into the nucleus of a cell and stably integrating said transgene in the genome of said cell, said vector comprising at least one immortalization molecule ;
- is capable of immortalizing a non-dividing cell or a slowly-dividing cell ;
- does not irreversibly modify the phenotype of interest of a cell which has been modified by said vector ;
- is capable of immortalizing a cell without irreversibly modifying the phenotype of interest of said cell.

In accordance with the present invention :

The term « vector » includes at least one nucleic acid molecule possibly assembled with at least one protein or peptide, said vector being capable of containing or comprising a transgene and of introducing said transgene into a target cell.

As used herein, the term "transgene" includes molecules that are not normally expressed in a cell.

The term « cell » means eukaryotic cell. The term « cell » includes cells of human or animal origin.

The term « molecule » includes a nucleic acid molecule, a peptide or a protein.

The term « nucleic acid molecule » includes DNA, cDNA, synthetic DNA, RNA, or combinations thereof.

The term « nucleic acid molecule » includes a DNA, a cDNA, a synthetic DNA, a RNA, or a combination thereof which is operably linked to a transcription regulatory molecule.

The term « transcription regulatory molecule » includes a promoter, an enhancer or a viral transcription regulatory molecule. Preferably, the transcription regulatory molecule is the Moloney murine leukemia virus promoter/enhancer element, the human cytomegalovirus enhancer or the vaccinia P7.5 promoter. In some cases, such as the Moloney murine leukemia virus promoter-enhancer element, these promoter/enhancer elements are located within or adjacent to the LTR sequences. Another preferred transcription regulatory molecule is the viral LTR promoter/enhancer signals. The transcription regulatory molecule can be homologous or heterologous to the desired gene sequence. Cell or tissue specific transcription regulatory molecules can be utilized to target expression of gene sequences in specific cell populations. Mammalian and viral promoters suitable for use in the practice of the present invention are well known and readily available in the art.

The term « nucleus » includes a body comprising chromosomes in an eukaryotic cell, said body being limited by the nuclear enveloppe.

As used herein, the term « non-dividing cells » can have the following definitions :
- cells which are blocked at various stages of the cell cycle. The various stages of the cell cycle in which the non-dividing cells can be blocked are the G0, G1, S, G2, prophase, prometaphase, and metaphase stage ;
- non-proliferating cells ;
- growth-arrested cells ;
- post-mitotic cells ;
- non-mitotic cells ;
- resting cells ;
- terminally-differentiated cells ;
- cells whose nuclear enveloppe is not broken ;
- primary cells ;
- non-tumoral cells ;
- non-treated cells.

Examples of non-dividing cells are primary cells, endocrine cells, endothelial cells, liver sinsusoidal endothelial cells, β-cells, hepatocytes, hematopoietic cells, stem cells, progenitor cells, neuronal cells, neuronal stem cells, lymphocytes, dentritic cells, epithelial cells, granulocytes and macrophages.

As used herein,the term « slowly-dividing cells » can have the following definitions :
- cells which are dividing slowly in vitro and which stop dividing without division-inducing treatment ;
- cells dividing more than 20, 24, 28, 32, 36, 40, 44, 48, 52, 56, 60, 64, 68, 72, 76, 80, 84, 88, 92 or 96 hours after their last division or after their last plating ;
- cells which cannot be infected, transduced and/or immortalized by an oncoretrovirus ;
- cells which cannot be infected, transduced and/or immortalized by an oncoretrovirus without modification of their phenotype of interest.

Examples of slowly-dividing cells are some primary cells, some specific myoblasts, some specific keratinocytes and some specific fibroblasts.

As used herein, the term « primary cell » can have the following definitions :
- cells obtained directly from an animal and which have not received any treatment or expansion ;
- cells present in a suspension of cells isolated from a vertebrate tissue source (prior to their being plate i.e., attached to a tissue culture substrate such as a dish or flask) ;
- cells present in an explant derived from tissue ;
- both of the two previous types of cells plated for the first time ;
- cell suspensions derived from the above-mentioned plated cells.

The expression « genetically modified » can mean infected, transfected, integrated and/or transduced.

An « immortalization molecule » can be a molecule which is capable of immortalizing at least one type of cell. A molecule which can immortalize a cell only in combination with at least one other immortalization molecule has to be essential to the immortalizing effect of said combination to be qualified as an « immortalization molecule » according to the invention. « Essential » can mean that the immortalization effect of the combination disappears if the immortalization molecule is suppressed of the combination.

An « immortalization molecule » can be a molecule that regulates negatively the expression of a gene which is responsible for the non-dividing or slowly-dividing state of a cell. The term "regulate negatively" can mean the suppression of expression of said gene. A gene which is responsible for the non-dividing or slowly dividing state of a cell can be a tumor suppressor gene. Where a non-dividing or slowly-dividing state of a cell is associated with the expression of a gene, an immortalization molecule can be a molecule that interferes with the gene's expression at the translational level. This approach utilizes, for example, antisense nucleic acid, ribozymes, or triplex agents to block transcription or translation of a specific mRNA, either by masking that mRNA with an antisense nucleic acid or triplex agent, or by cleaving it with a ribozyme. Antisense nucleic acids are DNA or RNA molecules that are complementary to at least a portion of a specific mRNA molecule (Weintraub, Scientific American, 262:40, 1990). In the cell, the antisense nucleic acids hybridize to the corresponding mRNA, forming a double-stranded molecule. The antisense nucleic acids interfere with the translation of the mRNA since the cell will not translate a mRNA that is double-stranded. Antisense oligomers of about 15 nucleotides are preferred, since they are easily synthesized and are less likely to cause problems than larger molecules when introduced into the target cell. The use of antisense methods to inhibit the in vitro translation of genes is well known in the art (Marcus Sakura, Anal.Biochem., 172:289, 1988). The triplex strategy uses an oligonucleotide to stall transcription. The oligomer winds around double-helical DNA, forming a three-strand helix. Triplex compounds can be designed to recognize a unique site on a chosen gene (Maher, et al., Antisense Res. and Dev., 1(3):227, 1991; Helene, C., Anticancer Druq Design, 6(6):569, 1991). Ribozymes are RNA molecules possessing the ability to specifically cleave other single-stranded RNA in a manner analogous to DNA restriction endonucleases. Through the modification of nucleotide sequences which encode these RNAs, it is possible to engineer molecules that recognize specific nucleotide sequences in an RNA molecule and cleave it. A major advantage of this approach is that, because they are sequence-specific, only mRNAs with particular sequences are inactivated.

The term « immortalization » can mean a method to obtain immortalized cells.

The term « immortalized cells » can have the following definitions :
- cells which are capable of growing indefinitely in culture due to the introduction of at least one « immortalization molecule » which confers altered growth properties upon the cell by virtue of expression of the immortalization molecule (s) within the genetically engineered cell ;
- cells having an indefinite longevity ;
- cells having an indefinite multiplication ;
- cells having an indefinite life-span ;
- cells that do not stop dividing if the condition of cultures are maintained ;
- cells that can be maintained in culture or in vitro during at least 25, 30, 35, 40, 45, 50 or 60 passages or during at least 100, 120, 140, 160, 180 or 200 population doublings or during at least 3, 4, 5, 6, 7 or 8 months ;
- cells that can be maintained in culture or in vitro during at least a number of passages, population doublings or a life-span which is two, three, four, five or ten times the normal number of passages, population doublings or life-span of said cells. The normal number of passages, population doublings or life-span of cells can mean their number of passages, population doublings or life-span if said cells are cultured without any agent capable of increasing the number of passages, population doublings or life-span of cells.

The term « phenotype of interest » of a specific cell includes phenotypes which interest the user of said specific cell. A « phenotype of interest » of a cell can vary with respect to the type of the cell and the subsequent use of said cell. A phenotype of interest of myoblasts is preferably their capacity of differentiation, more particularly their capacity of normal fusion to multinucleate myotubes.A phenotype of interest of keratinocytes is preferably their capacity to form normal epidermal equivalent in vitro. A phenotype of interest of endothelial cells is preferably the presence of LDL receptor and Von Willebrand factor, phagocytosis and fenestration.

More generally, a « phenotype of interest » of a cell is its original differentiation state, ie the differentiation state of this cell if this cell was non-dividing or slowly-proliferating when it was immortalized or the phenotype of interest of the ancestor of this cell which was non-dividing or slowly-proliferating when this ancestor was immortalized. If a cell is terminally-differentiated, a phenotype of interest of this cell can preferably be its differentiation state. If a cell is not completely differentiated (stem cells, progenitor cells, primary β cells), a phenotype of interest of this cell can preferably be its capacity of differentiation.

The phenotype of interest may be a phenotype naturally occuring for that cells, or alternatively may be a phenotype which results from the genetic modification of the cell. Such modification can occur before or after immortalization.

A vector of the invention is preferably defective.

In accordance with the present invention, the term « defective vector » can be a vector which is capable of entering a target cell but incapable of producing any viral particle capable of leaving this target cell or a vector that does not transform its target cell into a tumoral cell. The term « tumoral cell» includes cells that cause tumors when introduced into animals.

If the vector is a retrovirus, the term « defective retrovirus » can have the following definitions :
- a retrovirus that cannot be encapsidated in the target cell after transfection or infection of the target cell with this retrovirus,
- a retrovirus that do not include all of the viral structural proteins required for encapsidation or packaging of retroviral RNA into infectious virions,
- a retrovirus that do not include at least one of the viral structural factors required for encapsidation
- a retrovirus that has a cis defect which prevents encapsidation of genomic RNA.

In a preferred embodiment, a vector of the invention comprises at least one proliferation molecule, at least one anti-senescence molecule, at least one anti-apoptotic molecule and/or at least one molecule capable of modifying a differentiation pathway of a cell.

The expression « proliferation molecule » can have the following definitions :
- a molecule which is capable alone or in combination with another molecule to induce proliferation or division of a cell. A molecule which can induce proliferation or division of a cell only in combination with at least one other molecule has to be essential to the proliferation or division effect of said combination to be qualified as a « proliferation molecule » according to the invention. « Essential » can mean that the proliferation or division effect of the combination disappears if the proliferation molecule is suppressed of the combination ;
- a molecule which can induce entry into mitosis of a cell and/or stages of mitosis after entry into mitosis.

Preferred proliferation molecules are oncogenes,myc-like molecules (molecules functionally equivalent to myc), src-like (molecules functionally equivalent to src), SV40 large T antigen, adenovirus E1A, human papilloma virus E6 or E7, v-myc, Bmi-1, Src and ras.

A particularly preferred proliferation molecule is Bmi-1.

The term « anti-senescence molecule » includes molecules which prevent or reduce the attrition of the telomeres.

A preferred anti-senescence agent is telomerase.

Preferred anti-apoptotic molecules areBcl-2-like(molecules functionally equivalent to Bcl-2) and FLIP-like molecules(molecules functionally equivalent to FLIP).

Preferred anti-apoptotic molecules areBcl-2 and FLIP.

Preferably, a vector of the invention comprises at least one of the anti-apoptotic molecules of the Bcl-2 family and/or at least one of the anti-apoptotic molecules of the FLIP family.

Preferably, a vector of the invention comprises Bcl-2 and/or FLIP.

The term « a molecule capable of modifying a differentiation pathway of a cell » includes a molecule which is capable of delaying, arresting, modifying or accelerating the differentiation programm or differentiation pathway of a cell.

Preferred molecules capable of modifying a differentiation pathway of a cell are Notch receptors, especially the activated forms of the Notch receptors.

Preferably, a vector of the invention comprises one immortalization molecule.

Preferably, a vector of the invention comprises one proliferation molecule.

Preferably, a vector of the invention comprises at least one proliferation molecule and at least one anti-senescence molecule.

Preferably, a vector of the invention comprises Bmi-1 and telomerase.

Preferably, a vector of the invention comprises at least one proliferation molecule and at least one anti-apoptotic molecule.

Preferably, a vector of the invention comprises at least one proliferation molecule, at least one anti-senescence molecule and at least one anti-apoptotic molecule.

Preferably, a vector of the invention comprises Bmi-1, telomerase and Bcl-2.

Preferably, a vector of the invention comprises SV40 large T antigen, Bmi-1, telomerase and Bcl-2.

In a preferred embodiment, a vector of the invention comprises a regulator of transcription which is specific of the target cell type of said vector.

In a preferred embodiment, a vector of the invention comprises a system of deimmortalization of cells which have integrated said vector.

The term « deimmortalization » includes a method to suppress the immortalized phenotype of immortalized cells.

A preferred system of deimmortalization is a system allowing for the deletion of the immortalization molecules contained in a vector of the invention after said immortalization molecules have been integrated in the target cell.

A particularly preferred system of deimmortalization is the loxP/cre system of recombination/deletion.

In a preferred embodiment, a vector of the invention comprises a system allowing for the deletion of the immortalization molecules contained in said vector after said immortalization molecules have been integrated in the target cell.

In a preferred embodiment, a vector of the invention comprises at least one lox P site.

In a particularly preferred embodiment, a vector of the invention is a retrovirus.

Preferably, a retrovirus of the invention is a lentivirus.

The term « retrovirus » includes an RNA virus wherein the viral genome is RNA. When a host cell is infected with a retrovirus, the genomic RNA is reverse transcribed into a DNA intermediate which is integrated very efficiently into the chromosomal DNA of infected cells. This integrated DNA intermediate is referred to as a provirus.

In a particularly preferred embodiment, a vector of the invention comprises the following components : at least one component bearing at least one function of at least one protein of GAG, at least one component bearing at least one function of at least one protein of POL, a component allowing attachment and entry into the target cell of said vector, at least one element which is able to transport the nucleoprotein complex of said vector into the nucleus of the target cell of said vector and cis-acting elements necessary for reverse transcription and integration.

The above-mentioned components can be comprised in the retroviral genome and/or proviral DNA of the vector.

The above-mentioned components can be comprised in the proteins assembled with the retroviral genome.

The components bearing at least one function of at least one protein of GAG can be totally or partially derived from matrix, capsid, and/or nucleocapsid.

The components bearing at least one function of at least one protein of POL can be totally or partially derived from integrase and/or the RNA-directed DNA polymerase (reverse transcriptase).

A component allowing attachment and entry into the target cell of a vector of the invention is preferably a viral ENV.

A viral ENV is preferably amphotropic or ecotropic.

A viral ENV is preferably selected from the group consisting of Moloney leukemia virus (MLV) and Vesicular stomatitis virus (VSV) ENV.

An « element which is able to transport the nucleoprotein complex of said vector into the nucleus of the target cell of said vector » recited-above includes elements which associate with the nucleoprotein complex of said vector and are recognized by the nuclear import machinery of the target cell of said vector.

Elements which associate with the nucleoprotein complex of a vector(either directly or indirectly) and which are recognized by the nuclear import machinery of a target cell, as contemplated for use in the practice of the present invention include viral proteins which are directly recognized by the nuclear import machinery of a target cell, such as, for example, matrix protein (MA), integrase (IN), and the like, as well as viral proteins which are indirectly recognized by the nuclear import machinery of a non-dividing cell (by associating with the nucleoprotein complex and an agent which is recognized by the nuclear import machinery of target cell) , such as, for example, reverse transcriptase (RT), nucleocapsid, protease, and the like.

Preferably, an « element which is able to transport the nucleoprotein complex of said vector into the nucleus of the target cell of said vector » is selected from the group consisting of reverse transcriptase, matrix protein, nucleocapsid, protease, integrase and vpr.

A preferred « element which is able to transport the nucleoprotein complex of said vector into the nucleus of the target cell of said vector » is a lentiviral integrase, especially an HIV integrase.

Preferably, an « element which is able to transport the nucleoprotein complex of said vector into the nucleus of the target cell of said vector » comprises a nuclear localization signal (NLS).

Those of skill in the art can readily identify NLSs suitable for use herein. See, for example, the numerous NLS sequences described by Dingwall and Laskey in TIBS 16:478-481 (1991) and Goerlich and Mattaj in Science 271:1513-1518 (1996). For example, a suitable NLS can be derived from HIV-1 integrase. Another protein that exhibits karyophilic properties, and hence is useful herein, is Vpr. In addition, consensus NLSs have also been identified in the art, characterized as comprising a contiguous sequence of seventeen amino acids, wherein the first two amino acids are basic amino acids, followed by a spacer region of any ten amino acids, followed by a basic cluster in which at least three of the next five amino acids are basic. In addition, numerous specific NLSs have been identified in the art, e.g. : the amino acid sequence KRKQ (SEQ ID N°1 : Lys Arg Lys Gln), the amino acid sequence KELQKQ (SEQ ID N°2 : Lys Glu Leu Gln Lys Gln), the amino acid sequence KRKGGIG (SEQ ID N°3 : Lys Arg Lys Gly Gly Ile Gly), the amino acid sequence PKKKRKV (SEQ ID N°4 : Pro Lys Lys Lys Arg Lys Val), the amino acid sequence AAFEDLRVLS (SEQ ID N°5 : Ala Ala Phe Glu Asp Leu Arg Val Leu Ser), the yeast GAL4 targeting signal, and the like.

Preferably, a nuclear localization signal is derived from HIV-l integrase.

Preferably, an « element which is able to transport the nucleoprotein complex of said vector into the nucleus of the target cell of said vector » has undergone a modification or a mutation with respect to its natural structure so as to be recognized by the nuclear import machinery of the target cell.

Preferably, an « element which is able to transport the nucleoprotein complex of said vector into the nucleus of the target cell of said vector » is modified by the addition of a karyophilic component thereto.

Preferably, an « element which is able to transport the nucleoprotein complex of said vector into the nucleus of the target cell of said vector » is modified by the addition thereto of a nuclear localization signal.

Preferably, an « element which is able to transport the nucleoprotein complex of said vector into the nucleus of the target cell of said vector » is totally or partially derived from a lentivirus.

In a preferred embodiment, a vector of the invention comprises at least one karyophilic component.

A karyophilic component can be part of the matrix, vpr or integrase component of a lentivirus.

A karyophilic component can be a modified or mutated matrix, vpr or integrase component of a lentivirus.

The term « lentivirus » includes HIV, SIV, FIV, BIV, EIAV, VISNA or CAEV.

In another preferred embodiment, a vector of the invention comprises in its genome two further components which are two long terminal repeat (LTR) molecules.

The two long terminal repeat (LTR) molecules are preferably a 5' and 3' LTR respectively.

In a preferred vector of the invention, molecules necessary for reverse transcription of the genome (the tRNA primer binding site) and possibly also molecules necessary for efficient encapsidation of viral RNA into particles (the Ψ site)are adjacent to the 5' LTR.

The two long terminal repeat (LTR) molecules preferably flank the genome of a vector of the invention.

The LTRs serve to promote transcription and polyadenylation of the virion RNAs.

The LTRs can contain all other cis-acting molecules necessary for viral replication.

A LTR of a vector of the invention can comprise a site of recombination.

A preferred site of recombination which can be comprised in a LTR of a vector of the invention is a loxP site.

Preferably, a vector of the invention comprises at least one additional component which is chosen in the group consisting of vif, vpr, tat, rev, vpu, nef, and vpx.

The components of a vector of the invention recited-above are selected based on the type of cell targeted for introduction of said vector or for introduction of at least one immortalization molecule.

The components of a vector of the invention recited-above are preferably obtained from MoMuLV, HaMuSV, MuMTV, GaLV, RSV, HIV, SIV, FIV, BIV, EIAV, VISNA or CAEV.

The preferred components are obtained from a lentivirus and especially from HIV.

In another aspect, the invention provides a method of producing a vector of the invention recited above.

A method of producing a vector of the invention recited above can comprise introducing into a suitable packaging host cell one or more vectors comprising : a nucleic acid encoding at least one molecule having the function of at least one protein of GAG, a nucleic acid encoding at least one molecule having the function of at least one protein of POL, a nucleic acid encoding a component allowing attachment and entry into the target cell of said vector, a nucleic acid encoding an element which is able to transport the nucleoprotein complex of said vector into the nucleus of the target cell of said vector or an element which associates with the nucleoprotein complex of said vector and is recognized by the nuclear import machinery of the target cell, and a nucleic acid encoding a packaging signal flanked by cis-acting nucleic acids necessary for reverse transcription and integration, and a cloning site for introduction of at least one immortalization molecule, operably linked to a regulatory molecule.

A method of producing a vector of the invention recited above can comprise introducing into a suitable packaging host cell one or more vectors comprising : a nucleic acid encoding at least one molecule having the function of at least one protein of GAG, a nucleic acid encoding at least one molecule having the function of at least one protein of POL, wherein at least one protein encoded by said first or second nucleic acid is modified so as to be able to transport the nucleoprotein complex of said vector into the nucleus of the target cell or so as to be recognized by the nuclear import machinery of the target cell, a nucleic acid encoding a component allowing attachment and entry into the target cell of said vector, and a nucleic acid encoding a packaging signal flanked by cis-acting nucleic acids necessary for reverse transcription and integration, and a cloning site for introduction of at least one immortalization molecule, operably linked to a regulatory molecule.

A method of the invention can further comprise introducing at least one nucleic acid molecule encoding at least one molecule selected from the group consisting of vpr, vif, nef, vpx, tat, rev, and vpu.

A method of the invention can further comprise recovering the vectors produced by said transfected host cell.

In a preferred embodiment, a method of the invention produces a lentivirus-like virus and more particularly an human immunodeficiency virus-like virus.

A presently preferred method for the production of a vector of the invention involves the use of a combination of a minimum of three or four production vectors in order to produce a final vector of the invention as described above.

A first production vector provides a nucleic acid encoding at least one component having the function of at least one protein of GAG and at least one component having the function of at least one protein of POL.

The components bearing at least one function of at least one protein of GAG can be totally or partially derived from matrix, capsid, and/or nucleocapsid.

The components bearing at least one function of at least one protein of POL can be totally or partially derived from integrase and/or the RNA-directed DNA polymerase (reverse transcriptase).

Such components can be obtained from a variety of viral sources, e.g., Moloney murine leukemia virus (MoMuLV), Harvey murine sarcoma virus (HaMuSV), murine mammary tumor virus (MuMTV), gibbon ape leukemia virus (GaLV), human immunodeficiency virus (HIV), SIV, FIV, BIV, EIAV, VISNA, CAEV, Rous Sarcoma Virus (RSV), and the like.

A second production vector employed in the practice of the present invention provides a nucleic acid encoding a component allowing attachment and entry into the target cell of the final vector of the invention produced with said second vector.

A component allowing attachment and entry into the target cell of the final vector is preferably a viral envelope (env).

The viral envelope can be derived from any virus, including retroviruses. The env may be amphotropic envelope protein which allows transduction of cells of human and other species, or may be ecotropic envelope protein, which is able to transduce only mouse and rat cells.

Examples of retroviral-derived env genes include Moloney murine leukemia virus (MoMuLV), Harvey murine sarcoma virus (HaMuSV), murine mammary tumor virus (MuMTV), gibbon ape leukemia virus (GaLV), human immunodeficiency virus (HIV), SIV, FIV, BIV, EIAV, VISNA, CAEV, Rous Sarcoma Virus (RSV), and the like. Other env genes such as Vesicular stomatitis virus (VSV) (Protein G) can also be used.

The component allowing attachment and entry into the target cell of the final vector of the invention can advantageously be associated with an antibody or a particular ligand for targeting to a receptor of a particular target cell-type.

Thus, the second production vector can be made target specific by adding at least one sequence (including regulatory region) of interest into the second production vector.

A sequence of interest may code for a ligand of a receptor specific of a target cell.

The final vector can be made target specific by a glycolipid or a protein. Preferably, the final vector is made target specific by an antibody.

Those of skill in the art will know of, or can readily ascertain without undue experimentation, specific methods to achieve delivery of a vector of the invention to a specific target.

The nucleic acid encoding a component allowing attachment and entry into the target cell of the final vector of the invention is operably associated with regulatory sequence, e.g., a promoter or enhancer.

Preferably, the regulatory sequence is a viral promoter. The regulatory sequence can be any eukaryotic promoter or enhancer, including for example, the Moloney murine leukemia virus promoter-enhancer element, the human cytomegalovirus enhancer (as used in the illustrative example), or the vaccinia P7.5 promoter. In some cases, such as the Moloney murine leukemia virus promoter-enhancer element, these promoter-enhancer elements are located within or adjacent to the LTR sequences.

A third production vector contemplated for use in the practice of the present invention provides the cis-acting viral sequences necessary for the viral life cycle. Such sequences can include at least one of the sequences and signals contained in the group consisting of a Ψ packaging sequence, a reverse transcription signal, an integration signal, a viral promoter, an enhancer, and a polyadenylation sequence.

Such sequences or signals can be obtained from a variety of viral sources, e.g., MoMuLV, HaMuSV, MuMTV, GaLV, HIV, SIV, FIV, BIV, EIAV, VISNA, CAEV, RSV, and the like.

The third production vector also contains at least one cloning site for introduction of at least one heterologous nucleic acid sequence.

Within the context of the invention, the at least one heterologous nucleic acid sequence is at least one immortalization molecule as defined above and possibly at least one selection agent.

A preferred selection agent is HSV-TK.

Selectable marker genes can be utilized to assay for the presence of the vector, and thus, to confirm infection and integration. The presence of this marker gene ensures the growth of only those host cells which express the inserted DNA. Typical selection genes encode proteins that confer resistance to antibiotics and other toxic substances, e.s., histidinol, puromycin, hygromycin, neomycin, methotrexate, and the like. Other marker systems commmonly used in the art include S-galactosidase (LacZ) and luciferase reporter or marker systems, which can conveniently be monitored visually.

The third production vector is preferably bi- or poly-cistronic.

A preferred third production vector comprises an IRES sequence.

Optionally, a fourth production vector contemplated for use in the practice of the present invention provides the element which is able to transport the nucleoprotein complex of the final vector of the invention into the nucleus of the target cell of said vector or which associates with the nucleoprotein complex of said final vector.

A preferred element which is able to transport the nucleoprotein complex of the final vector of the invention into the nucleus of the target cell of said vector is HIV-1 integrase, particularly HIV-1 integrase expressed under the control of the HIV-1 promoter.

The method of the present invention described above contemplates the use of at least three vectors which provide all of the functions required for packaging of recombinant virions as discussed above, plus possibly a fourth vector which provides the element which is capable of associating with the nucleoprotien complex, thereby allowing immortalization of non-dividing or slowly-dividing cells.

The method of the present invention described above also contemplates transfection of vectors including viral genes such as vpr, vif, nef, vpx, tat, rev, and vpu. Some or all of these genes can be included, for example, on the packaging construct vector, or, alternatively, they may reside on production vectors. There is no limitation to the number of production vectors which can be utilized, as long as they are cotransfected to the packaging cell line in order to produce a single recombinant vector. For example, one could put the env nucleic acid sequence on the same construct as the gag and pol total or partial derivatives.

The production vectors contemplated for use herein are introduced via transfection or infection into a packaging cell line. The packaging cell line produces viral particles that contain the vector genome. Methods for transfection or infection are well known by those of skill in the art. After co-transfection of the at least three vectors to the packaging cell line, the recombinant virus is recovered from the culture media and titered by standard methods used by those of skill in the art.

The method for production of a vector of the invention described above employs three or more production vectors used to co-transfect a suitable packaging cell line. Since these production vectors collectively contain all of the required genes for production of a recombinant virus for infection and transfer of nucleic acid to a target cell, there is no need for use of a helper virus.

However, the first, second and/or fourth production vectors recited above can be replaced by using a helper cell line that provides the missing viral functions corresponding to these replaced vectors.

As readily recognized by those of skill in the art, a variety of different packaging cell lines can be prepared in accordance with the present invention. Thus, for example, a stable packaging cell line containing several of the above-described vectors can be prepared, such that one only need introduce a vector containing the heterologous nucleic acid sequence in order to produce a virion which is capable of producing a vector of the invention.

In a preferred embodiment, a vector of the invention is an HIV-based vectors which is achieved by transient co-transfection of 3 plasmids into 293T epithelial cell line as previously described (Naldini et al., 1996).

A first plasmid provides the envelope which is preferably the VSV-G envelope to pseudotype HIV particles because of its high stability and broad tropism.

A second plasmid (packaging plasmid) encodes for the structural and regulatory proteins of the virus. The second plasmid is preferably the R8.91 plasmid which encodes only for the HIV Gag and Pol precursors and the regulatory proteins Tat and Rev. In fact, other HIV proteins like Vpr, Vpu, Vif and Nef are dispensable for efficient infection of non-dividing cells (Zufferey et al., 1997). This provides an additional safety feature regarding possible recombination events in producer cells.

A third plasmid (vector plasmid) is designed for encapsidation in viral particles. Recent versions which are derivatives of the previously described pHR plasmid (Naldini et al., 1996)are preferably used. The most preferred versions are the latest versions which have a deletion in the U3 region of the 3'LTR leading to self-inactivation after reverse transcription (Zufferey et al., 1998)and/or which contain the post-transcriptional regulatory element of Woodchuck Hepatitis Virus (WHV-PRE)(Donello et al., 1998) which allows for greater RNA levels in both producer and target cells (Zufferey et al., 1999).

In another aspect, the invention provides a vector obtainable by any one of the method for producing a vector of the invention described above.

In another aspect, the invention provides a method for the immortalization of cells, said method comprising introducing into said cells at least one of the vectors of the invention described above and expressing the immortalization molecules encoded by said vector in said cells.

A method of immortalization of the invention as recited above is preferably carried out in vitro or ex vivo.

Alternatively, the method may be performed in vivo.

In a preferred embodiment, a vector of the invention used in a method of immortalization recited above is partially or totally derived from a retrovirus, especially a lentivirus.

Vectors of the invention are excellent vectors to expand non-dividing or slowly-dividing cells in vitro.

Preferred examples of cells which can be immortalized by a method of the invention are endothelial cells, myoblasts, keratinocytes, β-cells, hepatocytes, hematopoietic cells, stem cells, progenitor cells, neuronal cells, neuronal stem cells, lymphocytes, dendritic cells, epithelial cells, granulocytes and macrophages.

In another aspect, the invention provides a stable producing cell line capable of producing a vector of the invention recited above.

In an important aspect, the invention provides immortalized cells obtainable by a method according to any one of the methods of immortalization of the invention described above.

The invention provides cells originally non-dividing or slowly-dividing characterized in that they are immortalized.

The term « immortalized cells » can have the following definitions :
- cells which are capable of growing indefinitely in culture due to the introduction of at least one « immortalization molecule » which confers altered growth properties upon the cell by virtue of expression of the immortalization molecule(s) within the genetically engineered cell,
- cells having an indefinite longevity,
- cells having an indefinite multiplication,
- cells having an indefinite life-span,
- cells that do not stop dividing if the condition of cultures are maintained,
- cells that can be maintained in culture or in vitro during at least 25, 30, 35, 40, 45, 50 or 60 passages or during at least 100, 120, 140, 160, 180 or 200 population doublings or during at least 3, 4, 5, 6, 7 or 8 months,
- cells that can be maintained in culture or in vitro during at least a number of passages, population doublings or a life-span which is two, three, four, five or ten times the normal number of passages, population doublings or life-span of said cells. The normal number of passages, population doublings or life-span of cells can be their number of passages, population doublings or life-span if said cells are cultured without any agent capable of increasing the number of passages, population doublings or life-span of cells.

A « cell originally non-dividing or slowly-dividing » includes a cell or a descendant of a cell which was non-dividing or slowly-dividing when it was immortalized.

The invention provides cells which are immortalized and do not lose irreversibly their phenotype of interest.

The term « phenotype of interest » of a specific cell includes phenotypes which interest the user of said specific cell. A « phenotype of interest » of a cell can vary with respect to the type of the cell and the subsequent use of said cell. A phenotype of interest of myoblasts is preferably their capacity of differentiation, more particularly their capacity of normal fusion to multinucleate myotubes.A phenotype of interest of keratinocytes is preferably their capacity to form normal epidermal equivalent in vitro. A phenotype of interest of endothelial cells is preferably the presence of LDL receptor Von Willebrand factor, phagocytosis and fenestration.

More generally, a « phenotype of interest » of a cell is its original differentiation state, ie the differentiation state of this cell if this cell was non-dividing when it was immortalized or the phenotype of interest of the ancestor of this cell which was non-dividing when this ancestor was immortalized. If a cell is terminally-differentiated, a phenotype of interest of this cell can preferably be its differentiation state. If a cell is not completely differentiated (stem cells, progenitor cells, primary β cells), a phenotype of interest of this cell can preferably be its capacity of differentiation.

The invention provides cells which are immortalized and do not lose irreversibly their original phenotype of interest.

An « original phenotype of interest » of a cell can be the phenotype of interest of this cell if this cell is a primary cell or the phenotype of interest of primary ancestor of this cell.

The phenotype of interest may be a phenotype naturally occuring for that cells, or alternatively may be a phenotype which results from the genetic modification of the cell. Such modification can occur before or after immortalization.

The invention provides cells originally non-dividing or slowly-dividing and comprising integrated in their genome a provirus corresponding to a vector of the invention.

The invention provides cells which have not lost irreversibly their original phenotype of interest and which comprise integrated in their genome a provirus corresponding to a vector of the invention.

The invention provides immortalized cells comprising integrated in their genome a provirus corresponding to a vector of the invention.

Preferably, cells of the invention comprise in their genome at least one LTR site.

In a preferred embodiment, cells of the invention comprise two LTR sites in their genome.

Preferably, the two LTR sites are separated by at least one immortalization nucleic acid molecule operably linked to a transcription regulatory nucleic acid molecule.

The LTR sites above-mentioned can be of retroviral origin, preferably of lentiviral origin, and more preferably of HIV origin.

The LTR sites above-mentioned can comprise a site of recombination, preferably a loxP site.

Preferably, cells of the invention comprise integrated in their genome at least one selectable marker and/or at least one immortalization molecule. A preferred selectable marker is HSV-TK.

In a preferred embodiment, a selectable marker and at least one immortalization molecule are present in the genome of a cell of the invention.

Preferably, a selectable marker and at least one immortalization molecule are operably linked to form a bi- or poly-cistronic sequence or cassette.

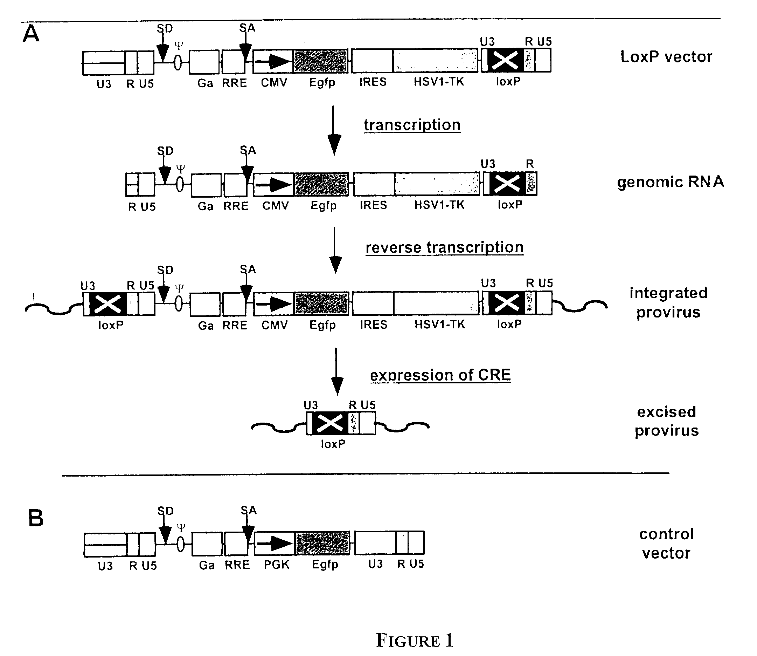

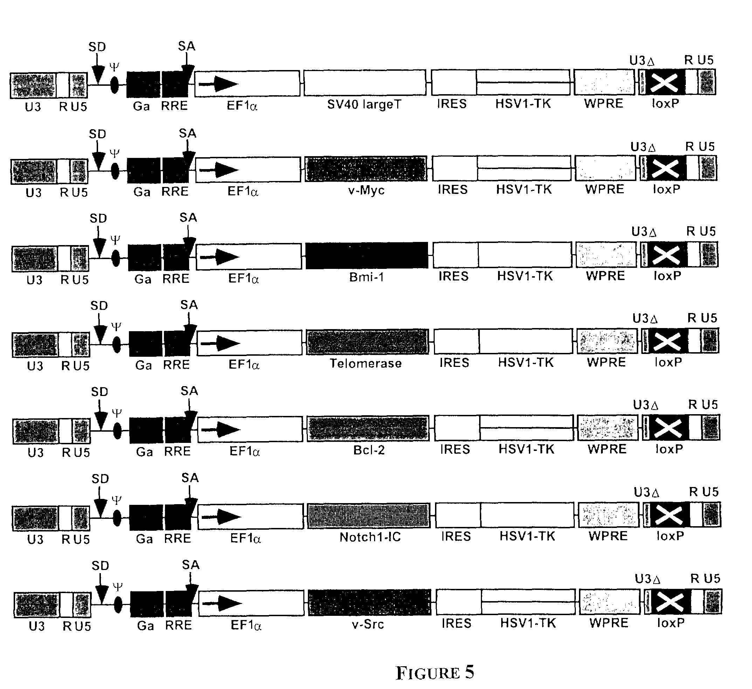

Preferably, a cell of the invention comprises all or part of at least one sequence depicted in Figure 1 or 5.

Preferably, cells of the invention are reversibly or conditionnally immortalized.

Preferably, cells of the invention recover their original phenotype of interest after deimmortalization.

Cells of the invention can be advantageously deimmortalized by a specific action.

A specific action can be to eliminate the foreign immortalization molecules.

The elimination of the foreign immortalization molecules is preferably carried out by putting in contact cells of the invention with a cre recombinase.

The phenotype of interest of cells of the invention can be the phenotype of a type of cell chosen in the group consisting of an endothelial cell, a myoblast, a keratinocyte, a β-cell, an hepatocyte, an hematopoietic cell, a stem cell, a progenitor cell, a neuronal cell, a neuronal stem cell, a lymphocyte, a dendritic cell, an epithelial cell and a macrophage.

Cells of the invention can be endothelial cells, myoblasts, keratinocytes, β-cells, hepatocytes, hematopoietic cells, stem cells, progenitor cells, neuronal cells, neuronal stem cells, lymphocytes, dendritic cells, epithelial cells and macrophages.

Cells of the invention are preferably not tumoral. The term « tumoral cell » includes cells that cause tumors when introduced into animals.

Cells of the invention can advantageously be used to produce a protein of interest in vitro.

In a preferred embodiment, cells of the invention are encapsulated.

Preferred cells of the invention are pancreatic β-cells.

The invention provides a bioartificial pancreas comprising pancreatic β-cells of the invention

Other preferred cells of the invention are keratinocytes.

The invention provides an equivalent epithelium or a skin comprising keratinocytes of the invention.

Keratinocytes of the invention can be used to treat burn or ulcers.

Preferred cells of the invention are liver sinusoidal endothelial cells.

Liver sinusoidal endothelial cells of the invention can advantageously be used for a pharmacological study.

Preferred cells of the invention are myoblasts.

The invention provides myotubes formed from the fusion of myoblasts of the invention.

Myotubes of the invention can advantageously be used to reconstitute muscles.

Preferred cells of the invention are dendritic cells.

Dendritic cells of the invention can advantageously be used to design anti-tumoral or anti-infectious substances.

Preferred cells of the invention are B-lymphocytes or plasmocytes.

B-lymphocytes or plasmocytes of the invention can advantageously be used to produce monoclonal antibodies.

### Figure legends

### Figure 1 :

### Schematic diagram of HIV/LOX vector life cycle.

The basic elements of HIV-based vectors, i.e. LTRs, SD, SA, Ψ, Ga, RRE, have been described (Naldini et al., 1996).
A. Life cycle of the HIV/LOX vector
B. Schematic diagram of the control HIV-based vector. The bicistronic cassette is replaced by a monocistronic cassette composed of the Egfp gene driven by the human phosphoglycerate kinase gene promoter (PGK). Both LTRs are intact.

### Figure 2 :

### Excision of HIV/LOX-gfp after transient transfection of Cre recombinase in HeLa cells.

Cells transduced with HIV vector or HIV/LOX vector (2x10⁶) were transiently co-transfected with 35 µg of a plasmid expressing NLS-Cre and 5 µg of a plasmid expressing murine CD8. Three days after transfection, cells were stained with PE-conjugated anti-mCD8 antibodies and analyzed by FACS. Cells were analyzed in 2-colors to gate for CD8-positive (transfected) and CD8-negative (untransfected) cells (CD8/Gfp dot plots, left panels) . Green (Gfp) fluorescence of gated CD8+ or CD8- cells was then displayed as histograms and percentage of Gfp-positive cells was determined.

### Figure 3 :

### Excision of HIV/LOX-gfp after transduction with adenoviral vectors containing Cre recombinase.

A stable clone of HeLa cells containing the HIV/LOXgfp construct (H4.5) was incubated with various doses of adenoviral vectors containing Cre recombinase (multiplicity of infection-MOI-ranging from 0.1 to 100). After 7 days, cells were analyzed by FACS and Gfp fluorescence was displayed as histograms and percentage of Gfp-positive cells was determined. Untreated H4.5 and parental HeLa were analyzed in paralel as positive and negative controls for Gfp fluorescence, respectively.

### Figure 4 :

### Residual marker-positive HeLa cells are efficiently eliminated after treatment with gancyclovir.

HeLa cells (upper left panel) were transduced with HIV/LOX-gfp vector. After 6 days, a bulk culture of HIV/LOX-gfp cells was analyzed by FACS (upper right panel) and transfected with Cre-expression plasmid (40 µg for 2x10⁶ cells). After 4 days, cells were analyzed by FACS (lower left panel) and subjected to treatment with 1 µM ganciclovir for 10 days. After treatment, cells were analyzed by FACS for residual gfp fluorescence.

### Figure 5 :

### Schematic representation of the HIV/LOX vectors used for reversible immortalization of human primary cells.

### Examples

### Example 1 : design of the excisable HIV-derived vector.

A HIV-based vector using the LoxP/Cre recombinase system (Hoess and Abremski, 1984; Sauer and Henderson, 1988)is used. This HIV vector contains the LoxP sequence in a SIN version of the 3'LTR (HIV/LOX vector). The schematic backbone of this vector is depicted in Figure 1. It contains the elements required for proper expression in producer cells (Naldini et al., 1996). The internal cassette is composed of two cistrons, including the gene coding for the enhanced green fluorescent protein (Egfp, Clontech Laboratories) as the reporter gene. This gene is replaced by the gene of interest (immortalization gene(s)) in the experiments of example 4. The second cistron is the thymidine kinase gene of Herpes simplex virus type 1 (HSV1-TK)(Colbere-Garapin et al., 1979), which is used as a conditional toxin. In one embodiment, the bicistronic cassette is under the transcriptional control of the immediate-early promoter of the human cytomegalovirus (Foecking and Hofstetter, 1986). Other promoters such as the EF1 promoter (human elongation factor EF-1-alpha gene) can be used to obtain high levels of expression in cells in which the CMV promoter is not efficient enough. Translation of HSV1-TK gene is driven by the internal ribosomal entry site of the Encephalomyocarditis virus (IRES)(Sugimoto et al., 1994). The LoxP sequence has been inserted in the 3' LTR, between the U3 and R sequences. The U3 sequence has been truncated in order to generate a self-inactivating LTR after reverse transcription and integration of the vector. Note that in the experiments described below, we used a version of HIV/LOX vector without WHV-PRE.

Virus particles are produced by the previously described three-plasmid transfection technique (Naldini et al., 1996). Genomic RNA borne by these viral particles is depicted in Figure 1. After reverse transcription and integration in target cells, the proviral DNA contains 2 copies of the LoxP sequence, flanking the entire bicistronic cassette as well as internal viral sequences. Then, upon transfection of a Cre bacterial recombinase expression plasmid in target cells, the DNA sequence present between the two LoxP sites is excised, leaving only one copy of the LoxP-inactivated LTR chimera in the host cell genome.

### Example 2 : transduction and excision of HIV/LOX in human cell lines.

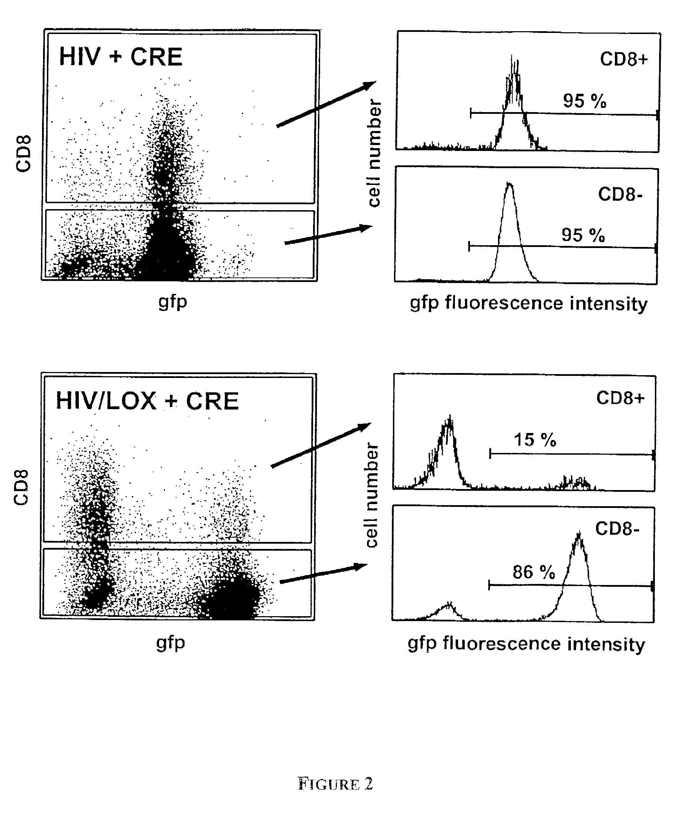

HeLa cells were infected with HIV/LOX-gfp (or control HIV-gfp) viral particles and individual clones were selected for high and homogeneous expression of gfp. The selected clones were then transiently co-transfected with an expression plasmid of the Cre bacterial recombinase tagged with a nuclear localization signal (NLS-Cre) together with a plasmid coding for a membrane marker not present on the surface of HeLa cells (i.e. murine CD8α), as a control for transfection. The ratio of Cre plasmid/CD8 plasmid was 7/1 to ensure for reliable expression of Cre in CD8-positive cells. Transfected cells were analyzed for both gfp and mCD8 expression 3 days after transfection. As shown in Figure 2, the percentage of Gfp-positive in CD8-positive (transfected) HIV/LOX-gfp cells decreases to 15 %, whereas CD8-negative (untransfected) cells remained 86 % Gfp-positive. It can be noted that co-transfection of Cre and mCD8 in cells infected with the control HIV-gfp vector did not alter the percentage of Gfp-positive cells (Figure 2), nor did the transfection of mCD8 alone in cells infected with HIV/LOX-gfp (data not shown). It can also be noted that similar results are obtained in independent clones of HeLa and 293T cells, as well as in bulk cultures of HeLa and 293T cells. These results indicate that the LoxP/Cre system is functional in the context of our HIV vectors, inducing a rapid loss of reporter expression after excision.

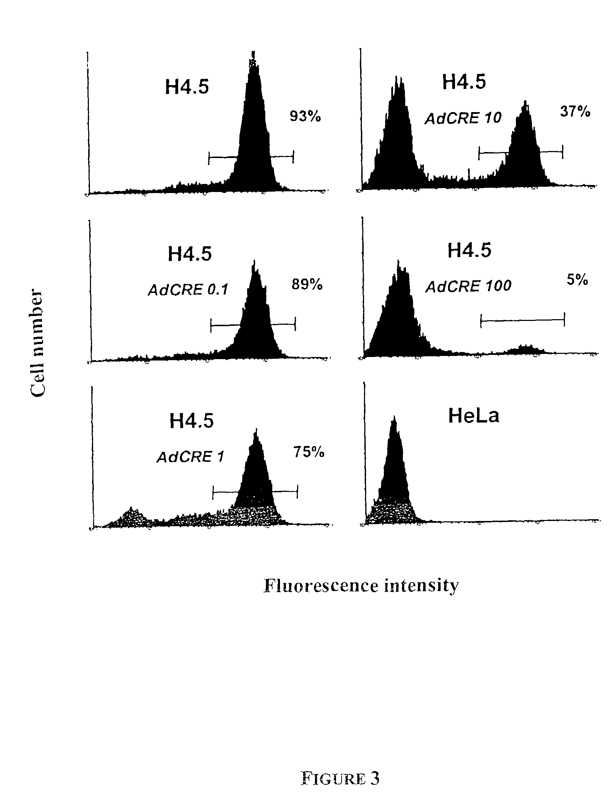

Similar results were obtained when Cre was delivered using adenoviral vectors (Figure 3). A stable clone of HeLa cells containing the HIV/LOX-gfp (clone H4.5) was incubated with various concentrations of particles of adenoviral vector coding for the Cre protein. As shown in Figure 3, the fraction of cells that remained Gfp+ after treatment with Ad-Cre vectors, was inversely proportional to the multiplicity of infection (MOI; 1 MOI represents 1 transducing unit per target cell). At a MOI of 100, 95% of the cells have excised the HIV/LOX-Gfp construct.

### Example 3 : elimination of residual gfp-positive cells using ganciclovir.

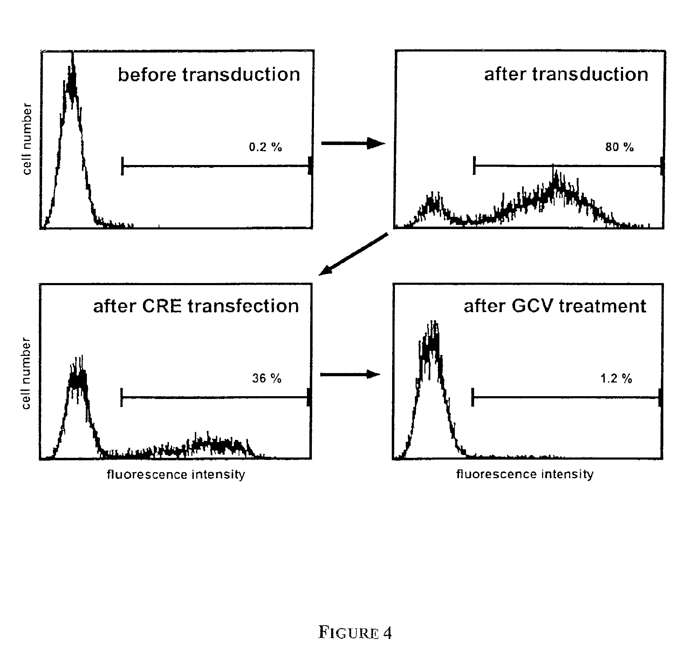

In order to be applicable for therapeutic purposes, reversible immortalization requires complete elimination of proliferating cells after the expansion phase. Even though Cre transduction will further be tested in a system more efficient than transfection (such as the use of high-titer Cre-adenovirus (Anton and Graham, 1995), we will most likely need to eliminate residual cells that would have escaped vector excision by Cre. We thus tested a conditional ablation system based on Herpes simplex virus type 1 thymidine kinase (HSV1-TK). Cells expressing the gene encoding for the HSV1-TK gene are sensitive to nucleoside analogs like acyclovir (ACV) or ganciclovir (GCV). These molecules are specifically converted by HSV1-TK into nucleotides that are toxic upon incorporation into DNA (Elion, 1983). This system thus specifically targets for cells which are both expressing HSV1-TK and dividing. To control for the efficacy of this system, we infected HeLa cells with HIV/LOX-gfp-IRES-TK vectors, and then went through the 2 sequential steps of vector elimination (Figure 4). Cells were first transfected with Cre plasmid to excise the vector. After 4 days, to allow for the excised vector to clear transfected cells, we added ganciclovir to the culture medium. As shown in Figure 4, a 10 day-treatment with standard concentration of ganciclovir achieves a decrease of Gfp-positive cells from 36 % to 1.2 % (30-fold reduction). Similar results were obtained in 293T cells, and using acyclovir. These results indicate that elimination of residual proliferating cells using the HSV1-TK system is efficient.

### Example 4 : immortalized human cells obtained using a retrovirus of the invention (HIV/LOX system).

Several immortalized cell lines have already been obtained by transducing primary human tissues with various immortalization cocktails. These include the three following examples :

### Liver sinusoidal endothelial cells (LSEC)

Liver sinusoidal endothelial cells do not normally proliferate in vitro.

These cells have been immortalized for the first time by transducing cells from a human liver biopsy with HIV/LOX vectors coding for SV40 large T-antigen and telomerase. These cells have been firstly infected with HIV/LOX vectors coding for SV40 large T-antigen and later infected with HIV-lox vectors coding for telomerase. The constructions used for transfection of SV40 large T antigen and telomerase respectively are depicted in figure 5.

These cells have been now maintained in culture for over 9 months (more than 60 passages).

Furthermore, these cells conserve their phenotype of interest, ie they display 3 features typical of LSEC, that is, i) expression of the LDL receptor and von Willebrand factor, ii) ability to perform phagocytosis and iii) fenestration.

These cells stop dividing after treatment with adenoviral vectors expressing Cre, and are otherwise sensitive to treatment with Ganciclovir. This represents the first example of immortalized liver sinusoidal endothelial cell line, which constitutes a powerful tool for a wide range of metabolic and pharmacological studies.This represents furthermore the first example of immortalized cells conserving their phenotype of interest.

Two clones of these cells named HL-SEC.E4 and HL-SEC.G9 have been deposited at the CNCM on November 25, 1999 under accession numbers I-2357 and I-2358.

### Myoblasts

The use of myoblasts/myotubes in ex vivo gene therapy protocols is limited by the insufficient numbers of primary myogenic cells that can be isolated from a muscle biopsy.

Primary clonal cultures were obtained from human muscle biopsies and infected with HIV/LOX vectors containing various combinations of the following genes : SV40 large T antigen, Bmi-1, Bcl-2 and telomerase. Whereas uninfected primary cell clones became senescent after 2 to 3 months or 9 passages in culture, as usually observed, we have obtained 4 cell lines that continue to proliferate after over 6 months in culture or after 45 passages. The combinations used to obtain these 4 immortalized lines are the following : SV40 large T antigen + telomerase, Bmi-1 + telomerase and SV40 large T antigen + telomerase + Bcl-2 + Bmi-1. The constructions used to transduce the myoblasts are depicted in figure 5. Amongst these 4 lines, one clone (transduced with Bmi-1 and telomerase) is capable of differentiating into multinucleated myotubes when shifted from «proliferation» into «differentiation» tissue culture medium, even without prior excision of the immortalizing genes. These cells are of value for the cell-based therapy of muscle disorders, and in situations where encapsulated cells are used to produce and release therapeutic proteins (i.e. erythropoietin, analgesic substances, etc.) into the body.

Two clones of these cells named HMB 6.13 and HMB 9.4 have been deposited at the CNCM on November 25, 1999 under accession numbers I-2355 and I-2356.

### Keratinocytes

Populations of human primary keratinocytes were obtained by explant culturing of the outer root sheath of hair follicles onto preformed fibroblast feeder layers. Subculturing was subsequently done onto collagen-coated dishes with a maximum of 3-5 passages for these primary cells. After transduction with HIV vectors containing the SV40 large T antigen / Bmi-1/Bcl-2/ telomerase genes, we have so far been able to culture these cells for at least 25 passages with continuous proliferation. The constructions used for transduction of keratinocytes are depicted in figure 5.

The differentiation status of these transduced cells before and after excision of the proviral sequences is currently being studied[. One immediate objective is to test the ability of these cells to reconstitute epidermal layers, with the long term goal of developing bioartificial skin for the treatment of chronic wounds and burns.

### References

Anton, M., and Graham, F. L. (1995). Site-specific recombination mediated by an adenovirus vector expressing the Cre recombinase protein: a molecular switch for control of gene expression. J. Virol. 69, 4600-4606.
Colbere-Garapin, F., Chousterman, S., Horodniceanu, F., Kourilsky, P., and Garapin, A. C. (1979). Cloning of the active thymidine kinase gene of herpes simplex virus type 1 in Escherichia coli K-12. Proc. Natl. Acad. Sci. USA 76, 3755-3759.
Donello, J. E., Loeb, J. E., and Hope, T. J. (1998). Woodchuck hepatitis virus contains a tripartite posttranscriptional regulatory element. J. Virol. 72, 5085-5092.
Elion, G. B. (1983). The biochemistry and mechanism of action of acyclovir. J. Antimicrob. Chemother. 12, 9-17.
Foecking, M. K., and Hofstetter, H. (1986). Powerful and versatile enhancer-promoter unit for mammalian expression vectors. Gene 45, 101-105.
Hoess, R. H., and Abremski, K. (1984). Interaction of the bacteriophage P1 recombinase Cre with the recombining site loxP. Proc. Natl. Acad. Sci. USA 81, 1026-1029.
Naldini, L., Blomer, U., Gallay, P., Ory, D., Mulligan, R., Gage, F. H., Verma, I. M., and Trono, D. (1996). In vivo gene delivery and stable transduction of nondividing cells by a lentiviral vector. Science 272, 263-267.
Naldini,L., Blömer,U ; Gage, F. H., Trono,D., and Verma I. M. (October 1996). Efficient transfer, integration, and sustained long-term expression of the transgene in adult rat brains injected with a lentiviral vector. Proc. Natl. Acad. Sci. USA 93, 11382-11388.
Sauer, B., and Henderson, N. (1988). Site-specific DNA recombination in mammalian cells by the Cre recombinase of bacteriophage P1. Proc. Natl. Acad. Sci. USA 85, 5166-5170.
Sugimoto, Y., Aksentijevich, I., Gottesman, M. M., and Pastan, I. (1994). Efficient expression of drug-selectable genes in retroviral vectors under control of an internal ribosome entry site. Biotechnology (NY) 12, 694-698.
Zufferey, R., Donello, J. E., Trono, D., and Hope, T. J. (1999). Woodchuck hepatitis virus posttranscriptional regulatory element enhances expression of transgenes delivered by retroviral vectors. J Virol 73, 2886-92.
Zufferey, R., Dull, T., Mandel, R. J., Bukovsky, A., Quiroz, D., Naldini, L., and Trono, D. (1998). Self-inactivating lentivirus vector for safe and efficient In vivo gene delivery. J Virol 72, 9873-80.
Zufferey, R., Nagy, D., Mandel, R. J., Naldini, L., and Trono, D. (1997). Multiply attenuated lentiviral vector achieves efficient gene delivery in vivo. Nat Biotechnol 15, 871-5.

## Claims

1. Vector capable of stably integrating a transgene in the genome of a non-dividing cell or of a slowly-dividing cell, said vector comprising or expressing at least one immortalization molecule.

2. Vector capable of transporting a transgene into the nucleus of a cell and stably integrating said transgene in the genome of said cell, said vector comprising at least one immortalization molecule.

3. Vector according to claim 1 or 2 characterized in that it is capable of immortalizing a non-dividing cell or a slowly-dividing cell.

4. Vector according to any one of claims 1 to 3 characterized in that it does not irreversibly modify the phenotype of interest of a cell which has been genetically modified by said vector.

5. Vector capable of immortalizing a cell without modifying the phenotype of interest of said cell.

6. Vector according to any one of claims 1 to 5, characterized in that it is defective.

7. Vector according to any one of claims 1 to 6, characterized in that at least one immortalization molecule is a proliferation molecule, an anti-senescence molecule, an anti-apoptotic molecule and/or a molecule capable of modifying a differentiation pathway of a cell.

8. Vector according to claim 7, characterized in that a proliferation molecule is chosen from the group consisting of an oncogene, SV40 large T antigen, adenovirus E1A, human papilloma virus E6 or E7, v-myc, Bmi-1, Src, ras.

9. Vector according to claim 8, characterized in that an anti-senescence molecule is telomerase.

10. Vector according to claim 8, characterized in that an anti-apoptotic molecule is chosen from the group consisting of Bcl-2 and FLIP.

11. Vector according to claim 5, characterized in that a molecule capable of modifying a differentiation pathway of a cell is a Notch receptor.

12. Vector according to any one of claims 1 to 11, characterized in that it comprises one immortalization molecule.

13. Vector according to claim 10, characterized in that the immortalization molecule is a proliferation molecule.

14. Vector according to any one of claims 1 to 13, characterized in that it comprises at least one proliferation molecule and at least one anti-senescence molecule.

15. Vector according to claim 14, characterized in that it comprises Bmi-1 and telomerase.

16. Vector according to any one of claims 1 to 15, characterized in that it comprises at least one proliferation molecule and at least one anti-apoptotic molecule.

17. Vector according to any one of claims 1 to 16, characterized in that it comprises at least one proliferation molecule, at least one anti-senescence molecule and at least one anti-apoptotic molecule.

18. Vector according to claim 17, characterized in that it comprises Bmi-1, telomerase and Bcl-2.

19. Vector according to claim 17, characterized in that it comprises Tag, Bmi-1, telomerase and Bcl-2.

20. Vector according to any one of claims 1 to 19, characterized in that a non-dividing or slowly-dividing cell is a non-human animal cell or a human cell.

21. Vector according to any one of claims 1 to 20, characterized in that a non-dividing cell is chosen from the group consisting of an endothelial cell, an endocrine cell, a β-cell, a hepatocyte, a hematopoietic cell, a stem cell, a progenitor cell, a neuronal cell, a neuronal stem cell, a lymphocyte, a dentritic cell, an epithelial cell and a macrophage.

22. Vector according to any one of claims 1 to 20, characterized in that a slowly-dividing cell is chosen from the group consisting of a myoblast or a keratinocyte.

23. Vector according to any one of claims 1 to 22, characterized in that it comprises a regulator of transcription which is specific to the target cell type of said vector.

24. Vector according to any one of claims 1 to 23 characterized in that it further comprises a system of deimmortalization of cells which have integrated said vector.

25. Vector according to any one of claims 1 to 24, characterized in that it comprises a system allowing for the deletion of the immortalization molecules contained in said vector.

26. Vector according to claim 25, characterized in that it comprises a lox P site.

27. Vector according to any one of claims 1 to 26 characterized in that it is a retrovirus.

28. Vector according to claim 27 characterized in that said retrovirus is a lentivirus.

29. Vector according to any one of claims 1 to 28 comprising the following components : at least one component bearing at least one function of at least one protein of GAG, at least one component bearing at least one function of at least one protein of POL, a component allowing attachment and entry into the target cell of said vector, an element which is able to transport the nucleoprotein complex of said vector into the nucleus of the target cell of said vector and cis-acting elements necessary for reverse transcription and integration.

30. Vector according to claim 29 wherein said element associates with the nucleoprotein complex and is recognized by the nuclear import machinery of the target cell of said vector.

31. Vector according to any one of claims 29 and 30 wherein said element is selected from the group consisting of reverse transcriptase, matrix protein, nucleocapsid, protease, integrase and vpr.

32. Vector according to any one of claims 29 to 31 wherein said element is a lentiviral integrase.

33. Vector according to claim 32 wherein said element is HIV integrase.

34. Vector according to any one of claims 29 to 33 wherein said element comprises a nuclear localization signal.

35. Vector according to claim 34 wherein said nuclear localization signal is derived from HIV-1 integrase.

36. Vector according to any one of claims 29 to 35 wherein said element has undergone a modification with respect to its natural structure so as to be recognized by the nuclear import machinery of the target cell.

37. Vector according to claim 36 wherein said element is mutated so as to be recognized by the nuclear import machinery of the target cell.

38. Vector according to any one of claims 29 to 37 wherein said element is modified by the addition of a karyophilic component thereto.

39. Vector according to any one of claims 1 to 38 comprising a karyophilic component.

40. Vector according to claim 38 or 39 wherein said karyophilic component is vpr, matrix protein or integrase.

41. Vector according to any one of claims 29 to 40 wherein said element is modified by the addition thereto of a nuclear localization signal.

42. Vector according to any one of claims 29 to 41, wherein a component allowing attachment and entry into the target cell of said vector is a viral ENV

43. Vector according to any one of claims 29 to 42, wherein the ENV is amphotropic or ecotropic.

44. Vector according to any one of claims 29 to 43, wherein the ENV is selected from the group consisting of Moloney leukemia virus (MLV) and Vesicular stomatitis virus (VSV) ENV.

45. Vector according to any one of claims 1 to 44, further comprising at least one viral protein selected from the group consisting of VPR, VIF, NEF, VPX, TAT, REV, and VPU.

46. Vector according to any one of claims 1 to 45, wherein a molecule is a nucleic acid molecule.

47. Vector according to claim 46, wherein the a nucleic acid molecule is operably linked to a transcription regulatory molecule.

48. Vector according to claim 47, wherein the is a transcription regulatory molecule is a promoter,an enhancer or a viral transcription regulatory molecule.

49. Vector according to any one of claims 1 to 48 wherein the components of said vector are selected based on the type of cell targeted for introduction of at least one immortalization molecule.

50. Vector according to any one of claims 1 to 49 wherein each or all of the components of said vector are obtained from MoMuLV, HaMuSV, MuMTV, GaLV, RSV, HIV, SIV, FIV, BIV, EIAV, VISNA or CAEV.

51. Method of producing a vector according to any one of claims 1 to 50, said method comprising introducing into a suitable packaging host cell one or more vectors comprising : a nucleic acid encoding at least one component bearing at least one function of at least one protein of GAG, a nucleic acid encoding at least one component bearing at least one function of at least one protein of POL, a nucleic acid encoding a component allowing attachment and entry into the target cell of said vector, a nucleic acid encoding an element which is able to transport the nucleoprotein complex of said vector into the nucleus of the target cell of said vector or an element which associates with the nucleoprotein complex of said vector and is recognized by the nuclear import machinery of the target cell, and a nucleic acid encoding a packaging signal flanked by cis-acting nucleic acids necessary for reverse transcription and integration, and a cloning site for introduction of at least one immortalization molecule, operably linked to a regulatory molecule.

52. Method according to claim 51 further comprising recovering the recombinant virus produced by said transfected host cell.

53. Method of producing a vector according to any one of claims 1 to 50, said method comprising introducing a suitable packaging host cell into one or more vectors comprising : a nucleic acid encoding at least one molecule having the function of at least one protein of GAG, a nucleic acid encoding at least one molecule having the function of at least one protein of POL, wherein at least one protein encoded by said first or second nucleic acid is modified so as to be able to transport the nucleoprotein complex of said vector into the nucleus of the target cell or so as to be recognized by the nuclear import machinery of the target cell, a nucleic acid encoding a component allowing attachment and entry into the target cell of said vector, a nucleic acid encoding a component allowing attachment and entry into the target cell of said vector, and a nucleic acid encoding a packaging signal flanked by cis-acting nucleic acids necessary for reverse transcription and integration, and a cloning site for introduction of at least one immortalization nucleic acid molecule, operably linked to a regulatory molecule.

54. Method according to claim 53 further comprising recovering the recombinant virus produced by said modified host cell.

55. Method according to any one of claims 51 to 54, wherein the vector is totally or partially derived from a lentivirus.

56. Method of claim 55, wherein the lentivirus is human immunodeficiency virus.

57. Method according to any one of claims 51 to 56, wherein the introducing further includes at least one vector providing a nucleic acid molecule selected from the group consisting of vpr, vif, nef, vpx, tat, rev, and vpu.

58. Recombinant vector obtainable by a method according to any one of claims 51 to 57.

59. Method for the immortalization of non-dividing or slow-dividing cells, said method comprising introducing into said non-dividing or slow-dividing at least one vector according to any one of claims 1 to 50, and expressing the immortalization molecules encoded by said vector(s) in said non-dividing or slow-dividing cells.

60. Method according to claim 59 wherein said cells are chosen from the group consisting of endothelial cells, myoblasts, keratinocytes, β-cells, hepatocytes, hematopoietic cells, stem cells, progenitor cells, neuronal cells, neuronal stem cells, lymphocytes, dendritic cells, epithelial cells, granulocytes and macrophages.

61. Method according to any one of claims 59 or 60, wherein the immortalization is carried out in vitro.

62. Method according to any one of claims 59 or 60, wherein the immortalization is carried out in vivo.

63. Method according to any one of claims 59 to 62, wherein the vector is totally or partially derived from a retrovirus or is totally or partially derived from a lentivirus.

64. A stable packaging cell line producing a vector according to any one of claims 1 to 50.

65. Immortalized cells obtainable by a method according to any one of claims 59 to 63.

66. Cells originally non-dividing or slowly-dividing characterized in that they are immortalized.

67. Cells characterized in that they are immortalized and do not lose irreversibly their original phenotype of interest.

68. Cells according to any one of claims 66 or 67 characterized in that they are reversibly or conditionally immortalized.

69. Cells according to claim 67 or 68 characterized in that they recover their original phenotype of interest after deimmortalization.

70. Cells according to claim 68 or 69 characterized in that they can be deimmortalized by a specific action.

71. Cells according to claim 70 wherein the specific action is to put in contact said cells with a cre recombinase or to eliminate the foreign immortalization molecules.

72. Cells originally non-dividing or slowly-dividing and comprising integrated in their genome a provirus corresponding to a vector according to any one of claims 1 to 50.

73. Cells which have not lost irreversibly their original phenotype of interest and comprising integrated in their genome a provirus corresponding to a vector according to any one of claims 1 to 50.

74. Immortalized cells comprising integrated in their genome a provirus corresponding to a vector according to any one of claims 1 to 50.

75. Cells according to claim 74 which are reversibly or conditionally immortalized.

76. Cells according to any one of claims 65 to 75, comprising in their genome at least one LTR site.

77. Cells according to claim 76 wherein one LTR site is of retroviral origin, such as of lentiviral origin, for example HIV origin, and optionally comprises a site of recombination and/or comprises a loxP site.

78. Cells according to any one of claims 73 to 75, comprising in their genome two LTR sites.

79. Cells according to claim 78 the two LTR sites are separated by at least one immortalization nucleic acid molecule operably linked to a transcription regulatory nucleic acid molecule.

80. Cells according to any one of claims 67, 69 and 73 characterized in that said phenotype of interest is the phenotype of a type of cell chosen in the group consisting of an endothelial cell, a myoblast, a keratinocyte, a β-cell, an hepatocyte, an hematopoietic cell, a stem cell, a progenitor cell, a neuronal cell, a neuronal stem cell, a lymphocyte, a dendritic cell, an epithelial cell and a macrophage.

81. Cells according to any one of claims 64 to 80 characterized in that they are chosen in the group consisting of endothelial cells, myoblasts, keratinocytes, β-cells, hepatocytes, hematopoietic cells, stem cells, progenitor cells, neuronal cells, neuronal stem cells, lymphocytes, dendritic cells, epithelial cells and macrophages.

82. Cells according to any one of claims 64 to 80 which are encapsulated.

83. Cells according to any one of claims 64 to 80 characterized in that they are pancreatic β-cells.

84. Cells according to any one of claims 64 to 80 characterized in that they are keratinocytes.

85. Cells according to any one of claims 64 to 80 characterized in that they are liver sinusoidal endothelial cells.

86. Cells according to any one of claims 64 to 80 characterized in that they are myoblasts.

87. Cells according to any one of claims 64 to 80 characterized in that they are dendritic cells.

88. Use of a vector according to any one of claims 1 to 50 to expand non-dividing or slowly-dividing cells in vitro.

89. Bioartificial pancreas comprising cells according to claim 83.

90. Skin comprising cells according to claim 84.

91. Use of cells according to any one of claims 64 to 80 to produce a protein of interest in vitro.

92. Use of cells according to claim 85 for a pharmacological study.

93. Myotubes formed from the fusion of cells according to claim 86.

94. Use of dendritic cells according to claim 87 to design anti-tumoral or anti-infectious substances.

95. Use of cells according to claim 84 to treat burn or ulcers.

96. Use of cells according to claim 86 or 93 to reconstitute muscles.

97. Cells according to any one of claims 64 to 80 characterized in that they are B-lymphocytes or plasmocytes.

98. Use of cells according to claim 97 to produce monoclonal antibodies.

99. Cells according to any one of claims 64 to 80 characterized in that they are not tumoral.

100. Process for the production of a protein of interest characterized in that immortalized cells according to any one of claims 66 to 99 are cultured in vitro or ex vivo, under conditions permitting the expression of said protein of interest in said cell, and optionally recovering said protein.

101. Process according to claim 100 wherein the protein of interest is endogenous to said cell.

102. Process according to claim 100 wherein said protein of interest is produced as a result of a genetic modification of said cell.

103. Process according to claim 102 wherein said genetic modification comprises the insertion into said cell, of a gene encoding said protein of interest, or the insertion of regulatory or coding sequences permitting expression of said protein.
